(19) **Europäisches Patentamt**
**European Patent Office**
**Office européen des brevets**

(11) **EP 2 328 551 B1**

(12) **EUROPEAN PATENT SPECIFICATION**

(45) Date of publication and mention of the grant of the patent:
**15.06.2016 Bulletin 2016/24**

(51) Int Cl.:
**A61K 9/00** *(2006.01)*          **A61K 9/10** *(2006.01)*
**A61K 47/38** *(2006.01)*

(21) Application number: **09792581.2**

(22) Date of filing: **16.09.2009**

(86) International application number:
**PCT/US2009/057065**

(87) International publication number:
**WO 2010/033528 (25.03.2010 Gazette 2010/12)**

(54) **STABILIZED PHARMACEUTICAL SUB-MICRON SUSPENSIONS AND METHODS OF FORMING SAME**

STABILISIERTE PHARMAZEUTISCHE SUBMIKRON-SUSPENSIONEN UND HERSTELLUNGSVERFAHREN DAFÜR

SUSPENSIONS SOUS-MICRONIQUES PHARMACEUTIQUES STABILISÉES ET LEURS PROCÉDÉS DE FORMATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.09.2008   US 98280 P**

(43) Date of publication of application:
**08.06.2011   Bulletin 2011/23**

(60) Divisional application:
**11191277.0 / 2 425 815**

(73) Proprietor: **Alcon Research, Ltd.**
**Fort Worth, TX 76134-2099 (US)**

(72) Inventors:
• **CASTILLO, Ernesto J.**
**Fort Worth**
**Texas 76140 (US)**
• **ASGHARIAN, Bahram**
**Arlington**
**Texas 76016 (US)**

• **CHOWHAN, Masood A.**
**Arlington**
**Texas 76016 (US)**

(74) Representative: **Kalhammer, Georg et al**
**Lederer & Keller**
**Patentanwälte Partnerschaft mbB**
**Unsöldstrasse 2**
**80538 München (DE)**

(56) References cited:
**EP-A- 0 592 348          WO-A-95/05804**
**WO-A-2005/032510     WO-A-2007/012974**

• **BOWEN P: "Particle Size Distribution Measurement from Millimeters to Nanometers and from Rods to Platelets" JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859 ISSN: 0193-2691**

**Description**

**Cross Reference to Related Application**

This application claims priority under 35 U.S.C. §119 to U.S. Provisional Patent Application No. 61/098,280, filed September 19, 2008.

**Technical Field of the Invention**

The present invention is related to a method of forming pharmaceutical submicron suspensions that employ low molecular weight polymers for stabilization. More specifically, the present invention relates to a method of forming pharmaceutical submicron suspensions that employ low molecular weight charged polymers for stabilizing a therapeutic agent while that agent is formed into submicron particles and/or while that therapeutic agent exists as submicron particles within the submicron suspension.

**Background of the Invention**

For many years, the pharmaceutical industry has been developing compositions that include therapeutic agents as well as systems and/or vehicles suitable for delivery of those therapeutic agents. In the ophthalmic, otic and nasal fields, a great deal of energy has been expended in developing fluid pharmaceutical compositions, particularly aqueous solutions, that include systems and/or vehicles suitable for delivery of therapeutic agents to the eye, ear or nose. During such development, many problems and difficulties can be encountered.

As one example, many therapeutic agents that exhibit desired therapeutic properties may also exhibit one or more properties that cause difficulty in developing pharmaceutical vehicles for delivering those agents. For instance, therapeutic agents can exhibit relatively high degrees of hydrophobicity and are formulated as suspensions, which can cause those agents to undesirably aggregate within an aqueous solution. In turn, the overall suspension can lack homogeneity and can, consequently, deliver inconsistent amounts of therapeutic agent to a target.

In efforts to accommodate these undesirable properties, many materials such as surfactants have been added to pharmaceutical vehicles with the objective of developing new stabilization systems. However, more recent discoveries have shown that many of these new systems can lack biocompatibility and can cause irritation or other undesirable effects to human tissue.

In further efforts to accommodate undesirable properties of therapeutic agents, ophthalmic, otic and nasal pharmaceutical compositions have been developed as suspensions. Suspensions can be particularly effective at accommodating therapeutic agents, which exhibit properties such as hydrophobicity, relative water insolubility or the like. However, therapeutic agents delivered as suspensions can also exhibit relatively poor therapeutic activity when delivered to target human tissue.

WO 2005/032510 discloses improved triamcinolone acetonide and anecortave acetate suspension compositions that are particularly suited for injection into the eye. The suspension compositions consist essentially of triamcinolone acetonide or anecortave acetate, polyvinylpyrrolidone, a tonicity-adjusting agent, a buffering agent and water for injection.

EP-A 0 592 348 refers to a pharmaceutical formulation in which two antibiotics and one anti-inflammatory drug are combined for topical use thereof in infectious eye and ear processes that are accompanied by inflammation. In particular, the pharmaceutical formulation comprises a combination of 2,4-diamino-5-(3',4',5'-trimethoxybenzyl) pyrimidine, known as trimethoprim, polyxymyxin, preferably polymyxin B and a steroidal anti-inflammatory drug, preferably dexamethasone or clobetasone, or a non-steroidal anti-inflammatory drug, preferably diclofenac or indomethacin, or a mixture of both types of anti-inflammatory drugs.

WO 95/05804 relates to ophthalmic vehicles and compositions which can be administered as a drop, but whose viscosity increases upon instillation into the eye so that the composition provides for relatively better drug delivery and duration of action of drug over aqueous compositions whose viscosity does not increase upon instillation. The vehicles comprise a charged polymer and oppositely charged electrolytes.

WO 2007/012974 discloses a drug delivery platform that can effectively deliver drugs, such as dexamethasone, to the posterior segments of the eye in therapeutically effective concentrations by topical administration to the eye. These ophthalmic compositions use micro- and nano-systems (micro-or nano-aggregates or solid micro- or nanoparticles), consisting of cyclodextrins, drugs and various exipients, which due to their size and/or surface charge, possess mucoadhesive properties that result in enhanced drug absorption into the eye.

One way to increase activity of a therapeutic agent is to increase the surface area of that agent. For example, it has been found that providing a therapeutic agent as submicron particles or nanoparticles can increase the surface area of the therapeutic agent and can exhibit significantly increased activity relative to that same therapeutic agent when it is provided as larger particles. It has also been found that such submicron particles can exhibit increased activity when delivered as part of submicron suspensions. However, formation of submicron suspensions can be difficult. For instance, it can be difficult to find suitable materials (e.g., milling agents) to assist in the formation of the submicron particles because such materials typically need to exhibit one or more desirable properties (e.g., wetting ability and/or low foaming) during submicron particle formation and will ultimately also need to exhibit one or more additional desirable properties (e.g., stability and/or biocompatibility) when they ultimately become part of the submicron suspension.

In view of the above, it would be desirable to provide a pharmaceutical submicron suspension (particularly a submicron ophthalmic suspension), a method of forming that suspension and/or materials suitable for forming that suspension, which overcome one or more of the aforementioned difficulties, problems and drawbacks

## Summary of the Invention

Accordingly, the present invention is directed to a process of forming a sub-micron suspension. According to the process, therapeutic agent is provided. The therapeutic agent has an original average hydrodynamic radius that is at least about 900 nanometers, more typically at least about 1.0 micron and even more typically at least about 1.3 microns and even possibly at least 2.0 microns, 4.0 microns or higher. The therapeutic agent is combined with a polymeric material to form an admixture. The polymeric material includes low molecular weight charged polymer and may be formed entirely or substantially entirely of low molecular weight charged polymer. The admixture is then processed to transform the particles of therapeutic agent into smaller submicron particles of therapeutic agent wherein the submicron particles of therapeutic agent have an average or mean hydrodynamic radius that is less than 1 micron, more typically no greater than 850 nanometers, still more typically no greater than 700 nanometers. Thereafter, the admixture becomes the sub-micron suspension preferably upon the addition of one or more excipients to the admixture and/or upon further processing. Advantageously, the low molecular weight charged polymer inhibits the aggregation of the particles and submicron particles during the processing and/or upon formation of pharmaceutical sub-micron nanosuspension.

In preferred embodiments, the therapeutic agent is a receptor tyrosine kinase inhibitor (RTKi) or a non-steroidal anti-inflammatory agent (NSAID) (e.g., nepafenac). Also is preferred embodiments, the low molecular weight charged polymer is substantially entirely or entirely carboxymethylcellulose.

## Detailed Description of the Invention

The method of the present invention is predicated upon the formation of a pharmaceutical composition and particularly a submicron suspension that includes a therapeutic agent in the form of submicron particles and includes a polymeric material (e.g., a charged polymer) that assists in stabilizing the therapeutic agent within the submicron suspension. The polymeric material can also be used to stabilize the therapeutic agent as relatively large particles of therapeutic agent are reduced to submicron or even nano- particles using one or more processing machines. It is contemplated that the pharmaceutical submicron suspension may be applicable in a variety of pharmaceutical contexts but can be particularly useful for otic and nasal applications. Thus, it is contemplated that the submicron suspension can be applied topically within the ear or nose of a mammal, particularly a human being. Most preferably, however, the submicron suspension is an ophthalmic suspension that can be applied topically or intravitreally to the eye of a human being.

As used herein the term "stabilize" and its conjugations as those terms are used in reference to the polymeric material stabilizing the therapeutic agent at least mean that the polymeric material inhibits the agglomeration of the particles of the therapeutic agent. As used herein, the term "submicron" as it is used to refer to particles means that the particles have a size that is less than one micron, however such particles can also have a size that is no greater than 850 nanometers and even possibly no greater than 700 nanometers. A submicron suspension is a suspension containing such particles suspended in solution. As used herein, the term "nanoparticle" means a particle having a size that is no greater than 200 nanometers, however such particles can also have a size that is no greater than 70 nanometers and even possibly no greater than 50 nanometers. A nanosuspension is a suspension containing such nanoparticles suspended in solution and a submicron suspension provided by the methods of the present invention can be a nanosuspension if the suspended particles are small enough.

Unless otherwise stated, particle size is determined by machine calculation. Several measurement machines are commercially available for measuring particle size within very small tolerances. Such machines measure particle size by, for example, dynamic light scattering and then calculate average or mean particle hydrodynamic radius for a set of particle. Those average or mean particle radii are, unless otherwise stated, the particle sizes discussed herein. One preferred exemplary machine is a ZETASIZER NANO, which is commercially available from Malvem Instruments Ltd., Enigma Business Park, Grovewood Road, Worcestershire WR14 1XZ, United Kingdom.

Measurement with particle sizing machines can require that certain parameters be provided to the machine prior to measurement of particle size in suspensions or other solutions. If required, parameters can be determined as follows: viscosity at zero shear rate ($\eta_0$) of a solution can be determined by oscillometer viscometer; refractive index of

the particles ($RI_p$) can be determined using the Becke Line Microscopic method; the refractive index of any diluent ($RI_d$) can be determined with a refractometer; and dielectric constant ($\kappa$) can be determined by capacitance measurements. As a general rule, it is preferable for the particle size measurement to be determined using solutions having relatively high concentrations of particles before multiple scattering and particle interactions affect the result.

Unless otherwise indicated, percentages provided for the ingredients of the pharmaceutical composition prepared by the methods of the present invention are weight/volume (w/v) percentages.

Therapeutic Agent

Typically, the submicron suspension provided by the methods of the present invention includes therapeutic agent. The therapeutic agent can be a single therapeutic agent or can be comprised of multiple therapeutic agents. Therapeutic agents include, but are not limited to, any component, compound, or small molecule that can be used to bring about a desired therapeutic effect. For example, a desired effect can include the cure, mitigation, treatment, or prevention of a disease or condition. A therapeutic agent can also affect the structure or function of a body part or organ in a subject.

Generally it is preferred that the therapeutic agent include at least one hydrophobic drug or therapeutic agent. A hydrophobic therapeutic agent includes an agent that is sparingly soluble in aqueous media (e.g., not completely dissolved in the media at the concentration at which it is administered in an aqueous composition) particularly when immersed in such aqueous media without aids to assist in solubilizing the agent. The therapeutic agent is typically at least about 0.001, more typically at least about 0.01 and still more typically at least about 0.1 w/v% of the submicron suspension. The therapeutic agent is typically less than about 10, more typically less than about 5 and still more typically less than about 2.0 w/v% of the submicron suspension.

The therapeutic agent employed according to the methods of the present invention is preferably a solid in particle form. However, it is also contemplated that therapeutic agent, such as therapeutic agent in liquid form, could be adsorbed by or otherwise disposed upon particles (e.g., polymeric particles) for use in the methods of the present invention.

A preferred class of therapeutic agents includes ophthalmic, otic and nasal drugs, particularly hydrophobic and/or low solubility ophthalmic, otic and nasal drugs. Non-limiting examples include: anti-glaucoma agents, anti-angiogenesis agents; anti-infective agents; anti-inflammatory agents; growth factors; immunosuppressant agents; and anti-allergic agents. Anti-glaucoma agents include beta-blockers, such as betaxolol and levobetaxolol; carbonic anhydrase inhibitors, such as brinzolamide and dorzolamide; prostaglandins, such as travoprost, bimatoprost, and latanoprost; seretonergics; muscarinics; dopaminergic agonists. Anti-angiogenesis agents include anecortave acetate (RETAANE™, Alcon™ Laboratories, Inc. of Fort Worth, Tex.) and receptor tyrosine kinase inhibitors. Anti-inflammatory agents include non-steroidal and steroidal anti-inflammatory agents, such as triamcinolone actinide, suprofen, diclofenac, ketorolac, nepafenac, rimexolone, and tetrahydrocortisol. Growth factors include EGF or VEGF. Anti-allergic agents include olopatadine and epinastine. The ophthalmic drug may be present in the form of a pharmaceutically acceptable salt.

The submicron suspension provided by the methods of the present invention has been found to be particularly desirable for ophthalmic applications (e.g., topical or intravitreal) when the therapeutic agent includes, is substantially entirely or is entirely receptor tyrosine kinase inhibitor (RTKi). Thus, in one preferred embodiment, the therapeutic agent can be at least 50%, more typically at least 80% and even more typically at least 95% (e.g., 100%) by weight RTKi.

The preferred RTKi for use in the present invention is a multi-targeted receptor tyrosine kinase inhibitor. Most preferred are RTKi's with multi-target binding profiles, such as N-[4-(3-amino-1H-indazol-4-yl) phenyl]-N'-(2-fluoro-5-methylphenyl) urea, having the binding profile substantially similar to that listed in Table 1 below. Additional multi-targeted receptor tyrosine kinase inhibitors contemplated for use in the compositions provided by the methods of the present invention are described in U.S. application Ser. No. 2004/0235892. As used herein, the term "multi-targeted receptor tyrosine kinase inhibitor" refers to a compound having a receptor binding profile exhibiting selectivity for multiple receptors shown to be important in angiogenesis, such as the profile shown in Table 1, and described in co-pending U.S. application Ser. No. 2006/0189608. More specifically, the preferred binding profile for the multi-targeted receptor tyrosine kinase inhibitor compounds for use in the compositions provided by the methods of the present invention is KDR (VEGFR2), Tie-2 and PDGFR.

TABLE 1

| Kinase Selectivity Profile of a RTK Inhibitor | | | | | | | | | | |
|------|------|------|-------|-------|-----|------|------|---------|---------|---------|
| KDR | FLT1 | FLT4 | PDGFR | CSF1R | KIT | FLT3 | TIE2 | FGFR | EGFR | SRC |
| 4 | 3 | 190 | 66 | 3 | 14 | 4 | 170 | >12,500 | >50,000 | >50,000 |

All data reported as IC50 values for kinase inhibition in cell-free enzymatic assays. Values determined @ 1 mM ATP.

Another highly preferred therapeutic agent suitable for use in suspensions provided by the methods of the present invention is, without limitation, a non-steroidal anti-inflammatory agent. The preferred non-steroidal anti-inflammatory agents are: prostaglandin H synthesis inhibitors (Cox I or Cox II), also referred to as cyclooxygenase type I and type II inhibitors, such as diclofenac, flurbiprofen, ketorolac, suprofen, nepafenac, amfenac, indomethacin, naproxen, ibuprofen, bromfenac, ketoprofen, meclofenamate, piroxicam, sulindac, mefanamic acid, diflusinal, oxaprozin, tolmetin, fenoprofen, benoxaprofen, nabumetome, etodolac, phenylbutazone, aspirin, oxyphenbutazone, NCX-4016, HCT-1026, NCX-284, NCX-456, tenoxicam and carprofen; cyclooxygenase type II selective inhibitors, such as NS-398, vioxx, celecoxib, P54, etodolac, L-804600 and S-33516; PAF antagonists, such as SR-27417, A-137491, ABT-299, apafant, bepafant, minopafant, E-6123, BN-50727, nupafant and modipafant; PDE IV inhibitors, such as ariflo, torbafylline, rolipram, filaminast, piclamilast, cipamfylline, CG-1088, V-11294A. CT-2820, PD-168787, CP-293121 DWP-205297, CP-220629, SH-636, BAY-19-8004, and roflumilast; inhibitors of cytokine production, such as inhibitors of the NFkB transcription factor; or other anti-inflammatory agents known to those skilled in the art. Preferred compounds for use as a prostaglandin synthesis inhibitor in the methods of the present invention are phenylacetamides selected from 2-Amino-3-(4-fluorobenzoyl)-phenylacetamide; 2-Amino-3-benzoyl-phenylacetamide (nepafenac); and 2-Amino-3-(4-chlorobenzoyl)-phenylacetamide, of which the most preferred is nepafenac.

The concentrations of the anti-inflammatory agents contained in the compositions provided by the methods of the present invention will vary based on the agent or agents selected and the type of inflammation being treated. The concentrations will be sufficient to reduce inflammation in the targeted ophthalmic, otic or nasal tissues following topical application of the compositions to those tissues. Such an amount is referred to herein as "an anti-inflammatory effective amount". The compositions provided by the methods of the present invention will typically contain one or more anti-inflammatory agents in an amount of from about 0.01 to about 3.0 w/v%, more typically from about 0.05 to about 1.0 w/v% and still more typically from about 0.08 to about 0.5 w/v%.

As suggested, it is preferable for the therapeutic agent (e.g., RTKi or NSAID such as nepafenac) suspended in the suspensions provided by the methods of the present invention to be hydrophobic. As such, the therapeutic agent typically has a log D that is greater than 0.1, more preferably greater than 0.4, more preferably greater than 0.6 and even possibly greater than 1.0 or even greater than 1.5.

As used herein, log D is the logarithm of the ratio of the sum of the concentrations of all forms of the therapeutic agent (ionized plus un-ionized) in each of two phases, an octanol phase and a water phase. For measurements of distribution coefficient, the pH of the aqueous phase is buffered to 7.4 such that the pH is not significantly perturbed by the introduction of the compound. The logarithm of the ratio of the sum of concentrations of the solute's various forms in one solvent, to the sum of the concentrations of its forms in the other solvent is called Log D:

$$\log D_{oct/wat} = \log \left( [\text{solute}]_{octanol} / \left( [\text{solute}]_{ionized\ water} + [\text{solute}]_{neutral\ water} \right) \right)$$

Other agents which may be useful in the methods of the invention include anti-VEGF antibody (i.e., bevacizumab or ranibizumab); VEGF trap; siRNA molecules, or a mixture thereof, targeting at least two of the tyrosine kinase receptors having $IC_{50}$ values of less than 200 nM in Table 1; glucocorticoids (i.e., dexamethasone, fluoromethalone, medrysone, betamethasone, triamcinolone, triamcinolone acetonide, prednisone, prednisolone, hydrocortisone, rimexolone, and pharmaceutically acceptable salts thereof, prednicarbate, deflazacort, halomethasone, tixocortol, prednylidene (21-diethylaminoacetate), prednival, paramethasone, methylprednisolone, meprednisone, mazipredone, isoflupredone, halopredone acetate, halcinonide, formocortal, flurandrenolide, fluprednisolone, fluprednidine acetate, fluperolone acetate, fluocortolone, fluocortin butyl, fluocinonide, fluocinolone acetonide, flunisolide, flumethasone, fludrocortisone, fluclorinide, enoxolone, difluprednate, diflucortolone, diflorasone diacetate, desoximetasone (desoxymethasone), desonide, descinolone, cortivazol, corticosterone, cortisone, cloprednol, clocortolone, clobetasone, clobetasol, chloroprednisone, cafestol, budesonide, beclomethasone, amcinonide, allopregnane acetonide, alclometasone, 21-acetoxypregnenolone, tralonide, diflorasone acetate, deacylcortivazol, RU-26988, budesonide, and deacylcortivazol oxetanone); Naphthohydroquinone antibiotics (i.e., Rifamycin).

Polymeric Material

Multiple polymers may be part of the polymeric material employed by the methods of the present invention. Examples of potentially suitable polymers include, without limitation, chondroitin sulfate, low molecular weight hyalouronic acid or other low molecular weight charged polymers that have a desired ability to lower or reduce surface tension. It is also contemplated that the submicron suspension provided by the methods of the present application could includes polymers in addition to or not included as part of the polymeric material. Examples of potentially suitable additional polymers include, without limitation, polyols, NIPAM polymers, polyethylene glycol, combinations thereof or the like.

Typically, however, the polymeric material will be comprised of one or more low molecular weight polymers, which are preferably charged. As used herein, the phrase "low molecular weight" as it is used to describe polymers of the polymeric material means that those polymers of the polymeric material cooperatively have an average molecular weight that is less than 200,000 and even more typically less than 100,000 kilodaltons (kDa). The viscosity of a solution of 1% polymeric material in purified water is typically at least 3.0, more typically at least 4.5 and still more typically at least 6.0 centipoise at 25°C and the viscosity of that solution is typically less than about 100, more typically less than about 20 and even more typically less than about 8.0 centipoise at 25°C.

Cellulose polymers such as carboxymethyl cellulose (CMC) polymers are particularly desirable for the polymeric material of the submicron suspension. As used herein, cellulose polymer includes any polymer that has two or more groups according to the formula ($C_6H_{10}O_5$). Such polymers can be charged when they are in salt form. Particularly desirable cellulose polymers are salts carboxymethyl cellulose polymer such as sodium carboxymethyl cellulose. Sodium carboxymethyl cellulose suitable for use in the present invention has a degree of substitution (DS) of at least 0.2 and preferably at least about 0.5. The degree of substitution of the sodium carboxymethyl cellulose can be up to about 2.5, preferably up to about 0.9. The degree of polymerization (DP) of the sodium carboxymethylcellulose is at least about 100, preferably at least about 200. The sodium carboxymethylcellulose degree of polymerization can be up to about 4,000, preferably up to about 1,000. One exemplary suitable cellulose polymer is sodium carboxymethyl cellulose is sold under the tradename AQUALON 7L2P and 7LF CMC, which is commercially available from Hercules Inc.

The submicron suspension provided by the methods of the present invention has been found to be particularly desirable for ophthalmic applications (e.g., topical or intravitreal) when the polymeric material includes, is substantially entirely or is entirely cellulose polymer (e.g., salt cellulose polymer such as sodium carboxymethylcellulose). Thus, the polymeric material can be at least 50%, more typically at least 80% and even more typically at least 95% (e.g., 100%) by weight cellulose polymer (e.g., salt cellulose polymer such as sodium carboxymethylcellulose).

Additional Ingredients

Various additional ingredients can be included in the sub-micron suspension of the present invention. The submicron suspensions provided by the methods of the present invention are typically aqueous and typically include a substantial amount (e.g., at least 80 or 90 w/v%) of water. The inclusion of other additional ingredients will typically depend upon how the submicron suspension is to be administered.

If the submicron suspension is to be administered topically to the eye or other human tissue, then the suspension can typically include a variety of additional ingredients. Such ingredients include, without limitation, additional therapeutic agents, antimicrobials, suspension agents, surfactants, tonicity agents, buffering agents, anti-oxidants, viscosity-modifying agents, any combinations thereof or the like.

If the submicron suspension is to be administered within the body, particularly intravitreally, by injection (e.g., needle) or otherwise, then it is typically desirable to minimize the amount of additional ingredients included in the submicron suspension. In such instance, it can be the case that the submicron suspension consists of or consists essentially of only the following ingredients: the polymeric material; the therapeutic agent and water.

Processing

The submicron suspension can be formed according to a variety of techniques within the scope of the present invention. According to a preferred protocol, the submicron suspension is formed using the following steps: i) the therapeutic agent is mixed as particles with the polymeric material and possibly excipients to form an admixture; ii) the admixture is supplied to a machine (e.g., a milling machine) that is configured to lower the particle size of the therapeutic agent; and iii) the admixture is combined with water and possibly excipients to form the submicron suspension.

The amounts of polymeric material and therapeutic agent in the admixture can vary and can depend upon the processing to be used for the admixture. However, it is generally preferable that the admixture be aqueous such that the polymeric material and therapeutic agent are added to water. In preferred embodiments, and particularly in embodiments that include a substantial portion of RTKi as therapeutic agent and a substantial portion of cellulose polymer as the polymeric material, the weight ratio of therapeutic agent to polymeric material is typically in a range from about 10:1 to about 1:10, more typically from about 5:1 to about 1:4 and even more typically from about 1.5:1 to about 1:1. In such embodiments, the admixture will typically include at least about 0.5, more typically at least about 1.5 and even more typically at least about 3.0 w/v% and will typically include less than about 12, more typically less than about 8 and even more typically less than about 4.0 w/v% therapeutic agent. Furthermore, in such embodiments, the admixture will typically include at least about 0.5, more typically at least about 1.2 and even more typically at least about 2.5 w/v% and will typically include less than about 12, more typically less than about 7 and even more typically less than about 3.8 w/v% polymeric material.

Examples of machines for lowering particle size include, without limitation, machines that perform high pressure homogenization and/or high-shear mixing. A preferred machine for lowering the particle size of the therapeutic agent is a wet milling machine. Such a machine can include a chamber filled with milling beads that are typically from about 0.05 mm to about 1 mm (e.g., 0.2 mm) in diameter. The chamber can then be rotated at a speed that is typically from about 2000 to about 4000 revolutions per minute (RPM). One exemplary suitable wet milling machine is a MINICER High Grinding System, commercially available from Netzsche Fine Particle Technology, Exton, PA, USA. It should be understood that the particles may need to be machined (e.g., milled) multiple times before the desired submicron or nanoparticle size is achieved.

The particles of the therapeutic agent, prior to machining or processing, typically have an average particle size that is greater than 500 nanometers, more typically greater than 1.0 microns and even more typically greater than about 1.3 microns. After machining or otherwise processing the particles, the particles either become submicron particles or become smaller submicron particles with a particles size that is less than about 900 nanometers, more typically less than about 820 nanometers and even more typically less than about 730 nanometers. For certain embodiments (e.g., for RTKi therapeutic agent), it may be desirable for the particle size of the therapeutic agent, after machining, to be greater than a particular size (e.g., nanoparticle size) to provide a therapeutic effect over an extended period of time for the agent. Thus, the submicron particles can have a size greater than about 200 nanometers, more typically greater than about 350 nanometers and even possibly greater than about 400 nanometers. In still other embodiments where it is desirable for the delivery of a greater therapeutic effect over a shorter period of time, it may desirable for the therapeutic agent to be still smaller after machining. In such embodiment, the submicron particles can have a particles size that is less than about 200 nanometers, more typically less than about 150 nanometers and even more possibly less than about 100 nanometers.

For other therapeutic agents, particularly NSAIDS such as nepafenac, the average particle size may be different. Such particle size is typically at least about 50 nanometers, more typically at least about 200 nanometers and even more typically at least about 250 nanometers. Such particle size is typically less than 820 nanometers, more typically less than 500 nanometers and even more typically less than 350 nanometers.

At some point before, during or after the processing of the therapeutic agent to achieve smaller particle size, excipients and/or active agents are added to the therapeutic agent, the polymeric material, the admixture thereof or a combination thereof for forming the submicron suspension. Thus, it is contemplated that excipients or additional active agents can be added before or after the polymeric material is combined with the therapeutic agent, before or after the particle size has been reduced or at any time during the processing. In a preferred step, the admixture is further diluted, preferably with purified water, after the desired particle size has been achieved such that final weight volume percents of polymeric material and therapeutic agent are achieved for the submicron suspension. At completion, the submicron suspension will typically include at least about 0.1, more typically at least about 0.5 and even more typically at least about 1.0 w/v% and will typically include less than about 7, more typically less than about 5 and even more typically less than about 2.5 w/v% therapeutic agent. Also, the submicron suspension will typically include at least about 0.1, more typically at least about 0.5 and even more typically at least about 1.0 w/v% and will typically include less than about 7, more typically less than about 5 and even more typically less than about 2.5 w/v% polymeric material.

Advantageously, the polymeric material employed by the methods of the present invention provides an aid to processing (e.g., machining such as milling) of the therapeutic agent into submicron particles and, at the same time, tends to inhibit aggregation of the particles of therapeutic agent and/or tends to exhibit a relatively low degree of foaming during such processing. Moreover, the polymeric material can act to inhibit aggregation of the submicron particles in the submicron suspension. Without being bound by theory, it is believed that the charge of the low molecular weight charge polymer assists in closely associating the polymer with the therapeutic agent, which is typically oppositely charged. In turn, this association is believed to assist in preventing agglomeration of the therapeutic agent. As an added advantage, the polymeric material employed by the methods of the present invention tends to be biocompatible.

Further, when an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range.

Other embodiments of the present invention will be apparent to those skilled in the art from consideration of the present specification and practice of the present invention disclosed herein. It is intended that the present specification and examples be considered as exemplary only with a true scope and spirit of the invention being indicated by the following claims.

**Experiments and Comparative Examples**

For determining the effect on aggregation of particles, a submicron suspension that included RTKi and Carboxy Methyl Cellulose (CMC) was prepared according to the teachings of the present invention. In particular, an admixture of water, CMC and RTKi were milled using a MicroCer Netzch High Energy Grinding System. Then, additional water was added to the admixture to form the submicron suspension as a 4 centipoise solution. The particle size within the submicron suspension was measured shortly after formation of the suspension. Thereafter, the submicron suspension was refrigerated and then the particle size was again measured at one week, six weeks and eight weeks after formation of the suspension. Each of these measurements was performed using a ZETASIZER NANO particle size measurement machine, commercially available from Malvern Instruments. The results are in Table A which are shown in conjunction with polydispersity index (Pd I) as follows:

TABLE A

| Storage Time | Particle | Size |
|---|---|---|
| | Z-average, nM | Pd I |
| Initial | 110 | 0.275 |
| 6 Weeks | 99 | 0.295 |
| 8 Weeks | 100 | 0.279 |
| 18 Weeks | 99 | 0.297 |
| 20 Weeks | 102 | 0.344 |

As can be seen, there is no significant changes in particle size. This suggests that littler or no agglomeration of particles has occurred at each of the time intervals. In particular, the measurement machine used to measure particle size produces larger particle size measurements when particles agglomerate. Since the measure of particle size in Table A remained substantially unchanged, the particles within the submicron suspension did not significantly agglomerate in the time intervals indicated. It should be noted that the particle size in Table A may not be exact depending on the accuracy of the inputs to the particle size measurement machine, however, the low degree of change in sizes is still quite reflective of the low agglomeration level since the inputs to the particle size measurement machine were consistent for subsequent measurements of the same solution.

Experiments were also performed on a variety of different potential milling aids and compared to CMC.

TABLE B

| Milling Aid % | Level of Foaming | Submicron suspension Homogeneity |
|---|---|---|
| Sodium CMC, 0.8 - 2.0% | Very Low | High Homogeneity |
| Polysorbate (PS) 80, 0.2% | Intermediate | High Homogeneity |
| Polystyrene Sulfonic Acid (PSSA), 4% | Low | Non-Homogeneous, Non-Wetting |
| PSSA 3.5%/PS 80 0.2% | Intermediate | High Homogeneity |
| Hyalouronic Acid, Na Salt, 0.7% | High | Non-Homogeneous |
| Polyvinyl pyrrolidone, 0.6% | Intermediate | Non-Homogeneous |

As can be seen, CMC minimizes foaming and provides for a desirable level of wetting.

For further determining the effect on aggregation of particles, Nepafenac and Carboxy Methyl Cellulose (CMC) were milled using a High Energy Grinding System. Then other ingredient were added to form the ophthalmic suspension in table C:

TABLE C

| Nepafenac | 0.3 |
|---|---|
| Sodium Carboxymethycellulose 7LF | 0.06 |
| Carbopol 974P | 0.5 |

(continued)

| Sodium Chloride | 0.4 |
|---|---|
| Propylene glycol | 1.1 |
| Benzalkonium Chloride | 0.01 |
| Disodium Edetate | 0.01 |
| NaOH/HCl | pH to 7.4 |
| Purified water | Qs to 100% |

The submicron suspension was monitored as a function of time up to 13 weeks at 25 and 40 °C. The particle size was evaluated by dynamic light scattering (Zatasizer) and reported below. The particle size is not siginificantly changed up to 13 weeks at either temperatures.

| | Storage Condition | Mean Particle Size (nm) |
|---|---|---|
| Initial | - | 586 |
| 4 week | 40 °C | 667 |
| 4 week | 25 °C | 643 |
| 13 week | 40 °C | 564 |
| 13 week | 25 °C | 565 |

## Claims

1. A method of forming an ophthalmic aqueous pharmaceutical submicron suspension, the method comprising:

providing a hydrophobic therapeutic agent in the form of particles wherein the particles have an average or mean hydrodynamic radius of at least 1 micron and wherein the therapeutic agent has a log D greater than 0.1; combining the particles of therapeutic agent with a low molecular weight charged polymer to form an admixture wherein the low molecular weight polymer includes one or more cellulose polymers that by itself or cooperatively have an average molecular weight that is less than 200,000 kilodaltons (kDa); processing the admixture to transform the particles of therapeutic agent into submicron particles of the therapeutic agent, the submicron particles of therapeutic agent having an average or mean hydrodynamic radius of less than 900 nanometers wherein the step of processing the admixture includes wet milling of the admixture; and combining the admixture with one or more excipients thereby forming the pharmaceutical submicron suspension wherein the low molecular weight charged polymer inhibits the aggregation of the particles and submicron particles during the processing or upon formation of the suspension; wherein log D is the logarithm of the ratio of the sum of the concentrations of all forms of the therapeutic agent (ionized plus un-ionized) in each of two phases, an octanol phase and a water phase; for measurements of distribution coefficient, the pH of the aqueous phase is buffered to 7.4 such that the pH is not significantly perturbed by the introduction of the compound; the logarithm of the ratio of the sum of concentrations of the solute's various forms in one solvent, to the sum of the concentrations of its forms in the other solvent is called log D.

2. A method as in claim 1 wherein the therapeutic agent is an RTKi or an NSAID.

3. A method as in claim 1 or 2 wherein the low molecular weight charged polymer is carboxymethylcellulose.

4. A method as in claim 1 or 2 wherein the low molecular weight charged polymer is sodium carboxymethylcellulose having a degree of substitution of at least 0.5 up to 0.9.

5. A method as in any of claims 1-4 wherein the therapeutic agent has a log D greater than 0.6.

6. A method as in any of claims 1-5 wherein the wet milling of the admixture occurs multiple times.

7. A method as in any of claims 1-6 wherein the one or more excipients include water.

8. A method as in any of claims 1-7 wherein the therapeutic agent has a log D greater than 1.0.

9. A method as in any of claims 1-8 wherein the suspension is an ophthalmic suspension suitable for administration to the eye.

10. A method as in any of claims 1-8 further comprising administering the suspension to an eye of a human as an intravitreal injection.

**Patentansprüche**

1. Verfahren zur Bildung einer ophthalmischen, wässerigen, pharmazeutischen Submikronsuspension, wobei das Verfahren umfasst:

das Bereitstellen eines hydrophoben Therapeutikums in Form von Teilchen, wobei die Teilchen einen durchschnittlichen oder mittleren hydrodynamischen Radius von zumindest 1 Mikrometer haben, und wobei das Therapeutikum einen log D größer als 0,1 hat;

das Vereinigen der Teilchen des Therapeutikums mit einem geladenen Polymer niederen Molekulargewichts unter Bildung eines Gemisches, wobei das Polymer niederen Molekulargewichts ein oder mehrere Cellulosepolymere umfasst, die selbst oder kooperativ ein durchschnittliches Molekulargewicht haben, das niedriger ist als 200.000 Kilodalton (kDa);

das Verarbeiten des Gemisches zur Umwandlung der Teilchen des Therapeutikums in Submikronteilchen des Therapeutikums, wobei die Submikronteilchen des Therapeutikums einen durchschnittlichen oder mittleren hydrodynamischen Radius von weniger als 900 Nanometern haben, wobei der Schritt der Verarbeitung des Gemisches das Nassmahlen des Gemisches umfasst; und

das Vereinigen des Gemisches mit einem oder mehreren Hilfsstoffen unter Bildung der pharmazeutischen Submikronsuspension, wobei das geladene Polymer niederen Molekulargewichts die Aggregation der Teilchen und Submikronteilchen während der Verarbeitung oder bei der Bildung der Suspension inhibiert, wobei log D der Logarithmus des Verhältnisses der Summe der Konzentrationen aller Formen des Therapeutikums (ionisierte plus nicht ionisierte) in jeder von zwei Phasen, einer Octanolphase und einer Wasserphase, ist, wobei für Messungen des Verteilungskoeffizienten der pH der wässerigen Phase auf 7,4 gepuffert wird, so dass der pH durch die Einführung der Verbindung nicht signifikant gestört wird, wobei der Logarithmus des Verhältnisses der Summe von Konzentrationen der verschiedenen Formen des gelösten Stoffes in einem Lösungsmittel zu der Summe der Konzentrationen seiner Formen in dem anderen Lösungsmittel als Log D bezeichnet wird.

2. Verfahren nach Anspruch 1, wobei das Therapeutikum ein RTKi oder ein NSAID ist.

3. Verfahren nach Anspruch 1 oder 2, wobei das geladene Polymer niederen Molekulargewichts Carboxymethylcellulose ist.

4. Verfahren nach Anspruch 1 oder 2, wobei das geladene Polymer niederen Molekulargewichts Natriumcarboxymethylcellulose mit einem Substitutionsgrad von zumindest 0,5 bis zu 0,9 ist.

5. Verfahren nach einem der Ansprüche 1 - 4, wobei das Therapeutikum einen log D größer als 0,6 hat.

6. Verfahren nach einem der Ansprüche 1 - 5, wobei das Nassmahlen des Gemisches mehrere Male stattfindet.

7. Verfahren nach einem der Ansprüche 1 - 6, wobei der eine oder die mehreren Hilfsstoff(e) Wasser umfasst/umfassen.

8. Verfahren nach einem der Ansprüche 1 - 7, wobei das Therapeutikum einen log D größer als 1,0 hat.

9. Verfahren nach einem der Ansprüche 1 - 8, wobei die Suspension eine ophthalmische Suspension ist, die zur Verabreichung an das Auge geeignet ist.

10. Verfahren nach einem der Ansprüche 1 - 8, ferner umfassend das Verabreichen der Suspension an das Auge eines Menschen als eine intravitreale Injektion.

**Revendications**

1. Procédé de formation d'une suspension submicronique pharmaceutique aqueuse ophtalmique, le procédé comprenant :

   la fourniture d'un agent thérapeutique hydrophobe sous la forme de particules dans lequel les particules ont un rayon hydrodynamique moyen d'au moins 1 micron et dans lequel l'agent thérapeutique a un Log D supérieur à 0,1 ;
   la combinaison des particules d'agent thérapeutique avec un polymère chargé à faible masse moléculaire pour former un mélange dans lequel le polymère à faible masse moléculaire inclut un ou plusieurs polymères de cellulose qui seuls ou conjointement ont une masse moléculaire moyenne qui est inférieure à 200 000 kilodaltons (kda) ;
   le traitement du mélange pour transformer les particules d'agent thérapeutique en particules submicroniques de l'agent thérapeutique, les particules submicroniques de l'agent thérapeutique ayant un rayon hydrodynamique moyen inférieur à 900 nanomètres dans lequel l'étape de traitement du mélange inclut un broyage humide du mélange; et
   la combinaison du mélange avec un ou plusieurs excipients formant ainsi la suspension submicronique pharmaceutique, le polymère chargé à faible masse moléculaire inhibant l'agrégation des particules et des particules submicroniques pendant le traitement ou lors de la formation de la suspension ; dans lequel
   Log D est le logarithme du rapport de la somme des concentrations de toutes les formes de l'agent thérapeutique (ionisées plus non ionisées) dans chacune des deux phases, une phase octanolique et une phase aqueuse; pour des mesures de coefficients de distribution, le pH de la phase aqueuse est tamponné à 7,4 de sorte que le pH ne soit pas perturbé de façon significative par l'introduction du composé; le logarithme du rapport entre la somme de concentrations des diverses formes du soluté dans un solvant, et la somme des concentrations de ces formes dans l'autre solvant est dénommé Log D.

2. Procédé selon la revendication 1, dans lequel l'agent thérapeutique est un RTKi ou un AINS.

3. Procédé selon la revendication 1 ou 2, dans lequel le polymère chargé à faible masse moléculaire est la carboxyméthylcellulose.

4. Procédé selon la revendication 1 ou 2, dans lequel le polymère chargé à faible masse moléculaire est la carboxyméthylcellulose de sodium ayant un degré de substitution d'au moins 0,5 jusqu'à 0,9.

5. Procédé selon l'une quelconque des revendications 1 à 4, dans lequel l'agent thérapeutique a un Log D supérieur à 0,6.

6. Procédé selon l'une quelconque des revendications 1 à 5, dans lequel le broyage humide du mélange a lieu de multiples fois.

7. Procédé selon l'une quelconque des revendications 1 à 6, dans lequel les un ou plusieurs excipients incluent l'eau.

8. Procédé selon l'une quelconque des revendications 1 à 7, dans lequel l'agent thérapeutique a un Log D supérieur à 1,0.

9. Procédé selon l'une quelconque des revendications 1 à 8, dans lequel la suspension est une suspension ophtalmique adaptée à une administration dans l'oeil.

10. Procédé selon l'une quelconque des revendications 1 à 8, comprenant en outre l'administration de la suspension dans un oeil d'un humain sous forme d'injection intravitréenne.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61098280 A
- WO 2005032510 A
- EP 0592348 A
- WO 9505804 A
- WO 2007012974 A
- US 20040235892 A
- US 20060189608 A